# EUROPEAN PATENT SPECIFICATION

(11) **EP 1 578 443 B1**
(45) Date of publication and mention of the grant of the patent: **12.01.2011**
(21) Application number: 03811387.4
(22) Date of filing: 20.11.2003
(51) Int. Cl.: A61K 39/39, A61K 9/107, A61K 39/145

(54) **COMPOSITIONS COMPRISING ANTIGEN-COMPLEXES, METHOD FOR MAKING SAME AS WELL AS METHODS OF USING THE ANTIGEN-COMPLEXES FOR VACCINATION**
ZUSAMMENSETZUNGEN MIT ANTIGEN-KOMPLEXEN,VERFAHREN ZU IHRER HERSTELLUNG UND VERFAHREN ZUR VERWENDUNG DERANTIGEN-KOMPLEXE ZUR VAKZINIERUNG
COMPOSITIONS COMPRENANT DES COMPLEXES D'ANTIGENES, LEUR PROCEDE DE FABRICATION AINSI QUE DES PROCEDES D'UTILISATION DESDITS COMPLEXES D'ANTIGENES POUR LA VACCINATION

(30) Priority: 20.11.2002 EP 02102610; 18.09.2003 WO PCT/EP03/50638
(43) Date of publication of application: 28.09.2005
(73) Proprietor: Bestewil Holding B.V., 2333 AL Leiden (NL)
(72) Inventor: STEGMANN, Antonius, Johannes, Hendrikus, NL-2224 AA Katwijk (NL)
(74) Representative: Jouannic, Nathalie
(86) International application number: PCT/EP2003/013084
(87) International publication number: WO 2004/045641

(56) References cited:
- EP-A- 0 231 039
- WO-A-00/70084
- WO-A-01/60402
- WO-A-02/00194
- WO-A-99/27954
- ANDO S ET AL: "Preparation of influenza virosome vaccine with muramyldipeptide derivative B30-MDP" JOURNAL OF MICROENCAPSULATION, vol. 14, no. 1, 1997, pages 79-90, XP000638910 ISSN: 0265-2048
- BAIER W ET AL: "Lipopeptides as immunoadjuvants and immunostimulants in mucosal immunization" IMMUNOBIOLOGY, vol. 201, no. 3/4, January 2000 (2000-01), pages 391-405, XP008020629 ISSN: 0171-2985 cited in the application

## Description

### FIELD OF THE INVENTION

The invention relates to vaccines directed against antigens from pathogens or tumor cells. The invention further relates to methods of forming specific complexes of antigens with amphiphilic compounds, and to pharmaceutical compositions comprising such complexes.

### BACKGROUND OF THE INVENTION

Classically, vaccines against enveloped viruses either contain killed or live attenuated viruses, or contain their membrane proteins (e.g. split virus preparation). After injection the virus particles or the proteins are taken up by cells of the immune system (for instance dendritic cells or macrophages) followed by a presentation of their antigenic parts to effector cells of the immune system. Most vaccines have to be infected to elicit a sufficiently strong immune response because antigen-presenting phagocytes are most abundant just under the skin. However, it has now become clear that such cells are also present in the mucosa that, for instance, lines the nose (Ogra et al. 2001). The phagocytes of the mucosa require much stronger stimulation than those present under the skin (Janeway et al. 2001).

While the injection of some viruses or proteins, for example influenza virus, elicits an immune response that is sufficiently strong to protect against a later infection by the same virus, this is not the case for many others, for example respiratory syncytial virus. Numerous attempts to reinforce the immune response by physical or chemical means (by compounds called adjuvants) have been undertaken. The most important principles that emerge from these experiments are as follows. For physical stimulation, it has been found that particles containing multiple copies of viral subunits, such as whole viruses, virosomes, and proteins on microparticle carriers stimulate the immune system better than individual subunits (Ogra et al. 2001; Janeway et al. 2001), whereas chemical stimulation requires that the phagocytes or the effector cells of the immune system receive certain signals through receptors present on the cell surface, for instance through the use of an adjuvant. With sufficient additional physicochemical stimulation, viral proteins can elicit strong immune responses even if applied to mucous membranes, for - example upon intranasal application (Ogra et al. 2001). Most of the current methods and compositions for stimulating an immune response by such means, whether by chemical or physical means or combinations of the two principles, have significant disadvantages that will be outlined below.

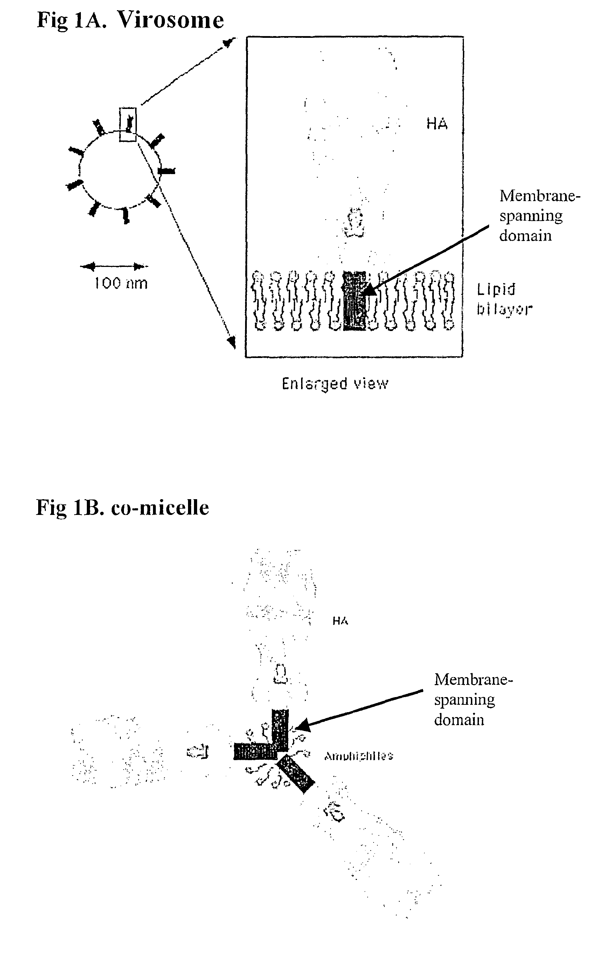

A particular kind of vaccine composition that was developed in the art is known as 'virosomes', which are lipid bilayers containing viral glycoproteins (Figure 1). Virosomes are generally produced by extraction of membrane proteins from viruses with detergents, followed by removal of the detergent in the presence of lipids such that characteristic lipid bilayers are formed with the proteins protruding from them (Stegmann et al. 1987). For some viral antigens, such virosomes elicit protective immune responses that are strong even when the vaccine is delivered through intranasal application (as is exemplified in WO 88/08718 and WO 92/19267). However, 30-85% of the viral proteins are lost during the process of virosome formation (WO 88/08718; Stegmann et al. 1987). Moreover, as the insertion of viral protein on either side of the membrane during reconstitution occurs with approximately equal probability, a large part of the protein (one third in the case of the influenza hemagglutinin, Stegmann et al. 1987) present in virosomes is present on the inside of the particles, and thus invisible to the immune system. Also, it is well known in the art that such artificial lipid bilayers render the preparation fragile, causing storage, handling and transport problems. Moreover, optimal formulation often requires complex mixtures of lipids, whose ratio has to be strictly controlled during production. This poses regulatory problems.

The immunogenicity of many viral antigens, for example influenza hemagglutinin, is only slightly improved with respect to killed viruses when the antigen is presented from virosomes (Gluck et al. 1994). Therefore, to enhance the immune response, allowing intranasal application of this vaccine, an adjuvant protein from Escherichia coli (heat-labile toxin) was mixed with the virosome influenza vaccine (EP 0538437). Clinical trials indicated that addition of the toxin was necessary to induce serum antibody titers equivalent to injected vaccine (Gluck et al. 1994). Although addition of the toxin did thus enhance the immunogenicity, it also induced a serious side effect known as Bell's Palsy, a temporary paralysis of facial muscles. In this case, the toxin did not form part of the virosomes, but was present in the surrounding solution. As the adjuvating effect of the toxin is due to recognition by an antigen presenting cell, enhancing its reaction to a viral protein that the cell might take up, and since there is no certainty that the toxin and the viral protein will contact the same cell, a relatively high concentration of the toxin needs to be used in order to ensure activation of every cell. Therefore, clearly, virosomes have promising features, such as their particulate nature, but a fair number of disadvantages.

Alternatively, researchers in the art have also generated antigen-complexes different from virosomes, such as 'Immunostimulatory Complexes' (ISCOMs, Morein et al. 1984), containing viral proteins complexed with compounds such as Quil A^{®} and saponins (EP 0231039B1; EP 0109942A1; EP 0180564A1), predominantly isolated from the bark of Quillaia sopanaria Molina. Mixed with antigen, and lipids such as cholesterol, these compounds form cage-like structures of between 30-40 nm, rendering the antigen particulate, while acting at the same time as an adjuvant. Although ISCOMs have been used in a number of veterinary vaccines, and enhance the immunogenicity of the viral membrane proteins, the development of such vaccines for humans has been inhibited by concerns about their toxicity and the complexity of the mixture (Cox et al. 1998).

More recently, proteosome influenza vaccines were developed (US application 20010053368), consisting of noncovalent complexes of the purified outer membrane proteins of bacteria such as meningococci, mixed with antigenic proteins such as the influenza hemagglutinin or the human immunodeficiency envelope glycoprotein. While the presence of these multiple bacterial proteins may act as an adjuvant, the complex nature of such mixtures, consisting of multiple proteins, will present a regulatory issue.

Another particulate formulation developed by Biovector Therapeutics consists of an inner core of carbohydrate surrounded by a lipid envelope containing antigens. With influenza hemagglutinin, as the antigen, some enhancement of the immune response was noted, but not significant enough to warrant further development.

Live attenuated versions of respiratory viruses, such as a cold-adapted strain of influenza virus with minimal replication in the respiratory tract have been developed as intranasal vaccines. These vaccines have the advantage of inducing immune responses that are close to the natural immunity induced by an infection with wild-type virus. For influenza, such vaccines have been known for 20 years, and now appear close to commercialization. The delay has been caused by concerns about the ability of many viruses to mutate rapidly, causing the properties of attenuated viruses to revert partially or wholly to wild-type virus, and in fact causing the disease they were meant to prevent.

WO 99/27954 describes the reparation of micellar compounds of a lipopetidic adjuvant and lipopeptides bearing specific epitopes. These lipopetides contain a lipid 'motif' next to their proteinaceous art which would harbour the epitopes (such as a palmitic acid moiety at the N-terminus).

Ando et al. (J. Microencapsulation 14(1):79-90, 1997)teaches virosome-like particles from a muramyl adjuvant compound (B30-DMP). However, this co-micelle structure has the same disadvantages as the previously known virosomes in that the epitopes of the antigen can be hidden in the lipid bilayer formed by the virosome.

For the above reasons, it is well recognized in the art that there still is a need for new vaccine compositions that induce a strong immune response, that do not have the disadvantages of live virus vaccines, that are easily applicable and have low toxicity. It is therefore recognized in the art that there is a need for sub-unit vaccines for intranasal delivery.

### DESCRIPTION OF THE FIGURES

Figure 1 shows a representation of the difference in size and composition between the antigen presenting complexes of the present invention (B) and virosomes (A); virosomes contain a viral integral membrane protein such as the influenza hemagglutinin, in a lipid bilayer, while the antigen presenting complexes of the present invention are co-micelles (not bilayers) of amphiphilic adjuvants with antigens such as the influenza hemagglutinin. In both cases, the membrane-spanning domain of the antigen is present in a hydrophobic environment: the interior of the lipid bilayer in the case of the virosomes, and the center of the micelle in the case of the co-micelles of the present invention. Thus, both types of particles expose at least that part of the protein to an aqueous environment, which can elicit an antibody response, and can be recognized on the viral membrane by the generated antibodies.

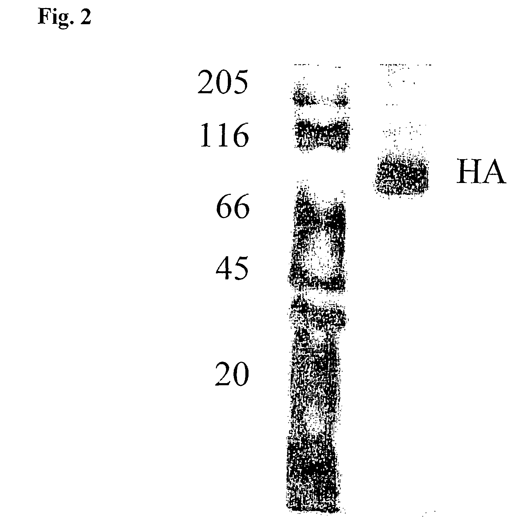

Figure 2 shows the purification of the hemagglutinin of influenza virus in a silver-stained non-reducing SDS-PAGE gel; left lane molecular weight standards with their apparent molecular weight in kilodaltons, right purified hemagglutinin.

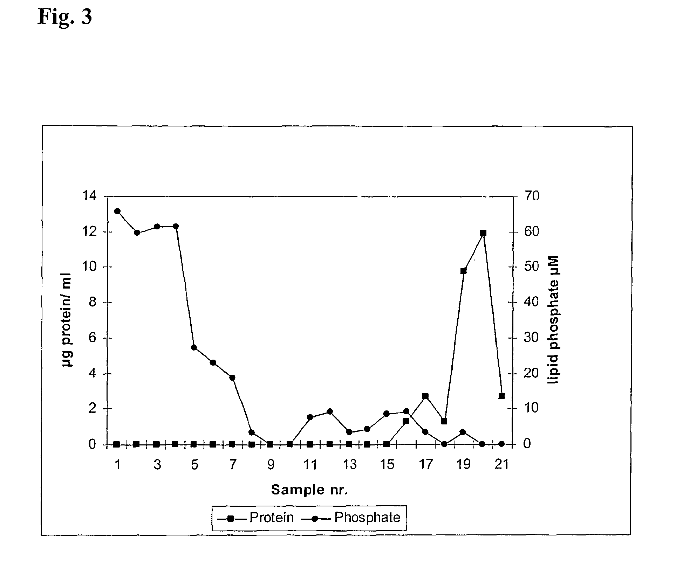

Figure 3 shows the delipidation of influenza A/new Caledonia 20/99 membrane proteins on a DEAE column; samples 1-10 buffer A, 11-15, buffer D, 15-20 buffer C. Indicated are phospholipid phosphate- and protein concentrations in the samples; samples 19 and 20 are pooled and used to produce the co-micelles of example 2.

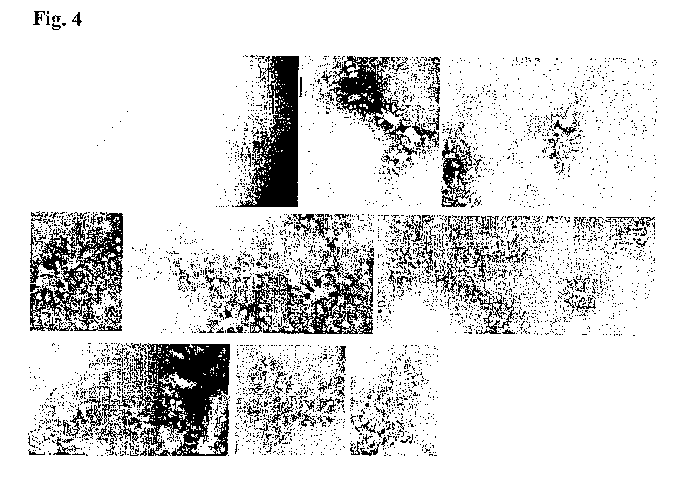

Figure 4 is a composition of electron micrographs of samples of co-micelles formed from influenza A/New Caledonia and N-palmitoyl-S-2,3(bispalmitoyloxy)-propyl-cysteinyl-seryl-(lysyl)₃-lysine, at a 1:1 weight ratio, stained with phosphotungsten acid (1% aqueous solution); clearly visible are the hemagglutinin proteins (spikes) protruding from a central core of lipopeptides. In several cases the influenza hemagglutinin protein projects towards the viewer, allowing the triangular ectodomain to be distuiguished, and indicating the central location of the membrane-spanning domain (example of triangular domains indicated by an arrow; the co-micelle that these domains are projecting from is below the plane of focus).

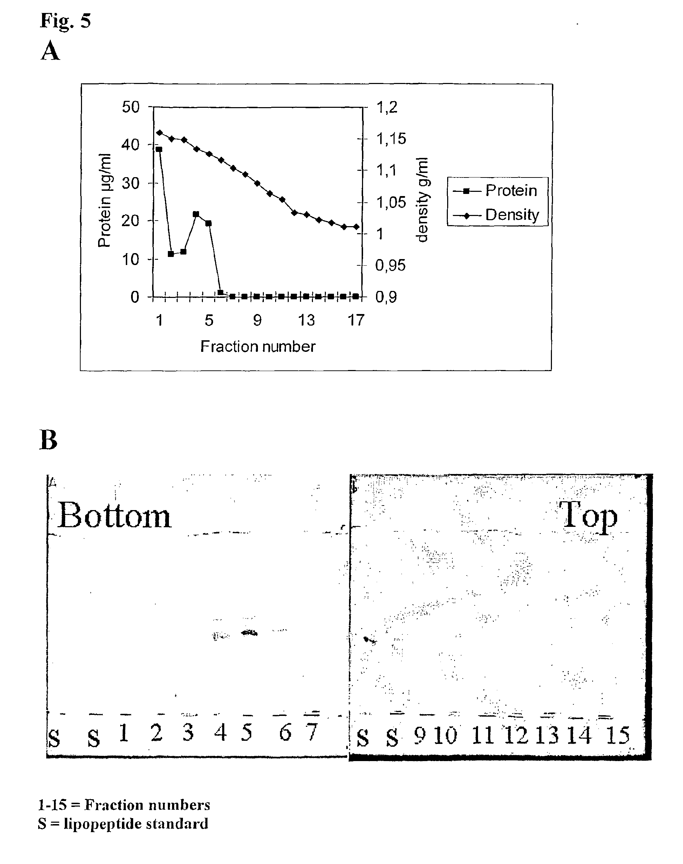

Figure 5 consists of a top panel (A) showing the density of fractions of a sucrose gradient, run until equilibrium in an ultracentrifuge, and the protein contents of these fractions, and a lower panel (B) consisting of two ninhydrin-stained thin layer chromatograms of the different fractions from the gradient (and a lipopeptide standard S), showing the physical association of the lipopeptide and the protein in a co-micelle particle.

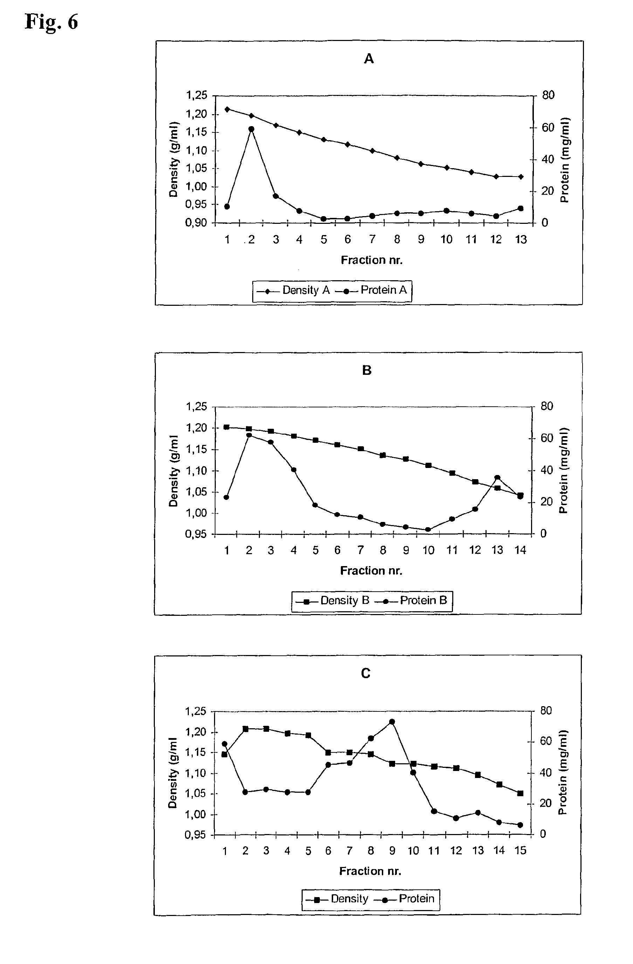

Figure 6 shows the protein concentration and density of fractions from sucrose gradients such as in Figure 4, for different protein to lipopeptide ratios: panel (A) at a 14:1, panel (B) 3:1, panel (C) 1:2; Notice the presence of non-incorporated viral protein in fraction 1 in panel (C).

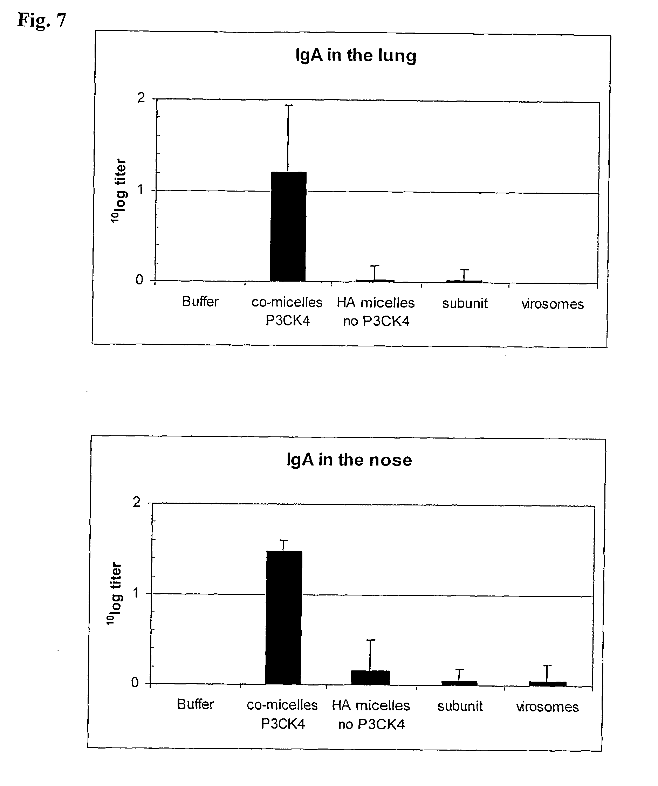

Figure 7 shows the geometric mean titers and standard error of the mean for IgA in groups of ten mice, that were immunized twice, with an interval of two weeks between immunizations, intranasally with either buffer, co-micelles of A/Panama proteins complexed with N-palmitoyl-S-2,3(bispalmitoyloxy)-propyl-cysteinyl-seryl-(lysyl)₃-lysine (abbreviated as P3CK4), micelles of influenza proteins without the lipopeptide (HA micelles no P3CK4), a standard influenza subunit vaccine (monovalent A/Panama), or virosomes prepared from A/Panama virus as described in Stegmann et al.(1987), in nose (lower panel) or lung (upper panel) washes. Samples were taken three weeks after the second immunization. The differences between the titers obtained after vaccination with co-micelles and all other groups are highly significant.

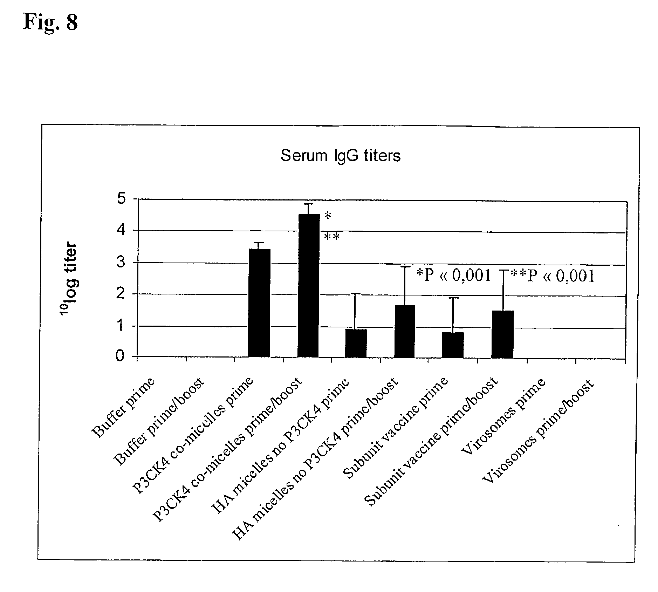

Figure 8 shows the geometric mean titers and standard error of the mean for serum IgG-in groups of ten mice, that were immunized once (prime), or twice (prime/boost) with an interval of two weeks between immunizations, intranasally with either buffer, co-micelles of A/Panama proteins complexed with N-palmitoyl-S-2,3(bispalmitoyloxy)-propyl-cysteinyl-seryl-(lysyl)₃-lysine (abbreviated as P3CK4), micelles of influenza proteins without the lipopeptide (HA micelles no P3CK4), a standard influenza subunit vaccine (monovalent A/Panama), or with virosomes in a prime and in a prime/boost set up. Samples were taken two weeks after the first or three weeks after the second immunization. The differences between the titers obtained after vaccination with co-micelles and all other groups are highly significant; for the two groups indicated with asterisks, p was « 0.001 by Student's t-test.

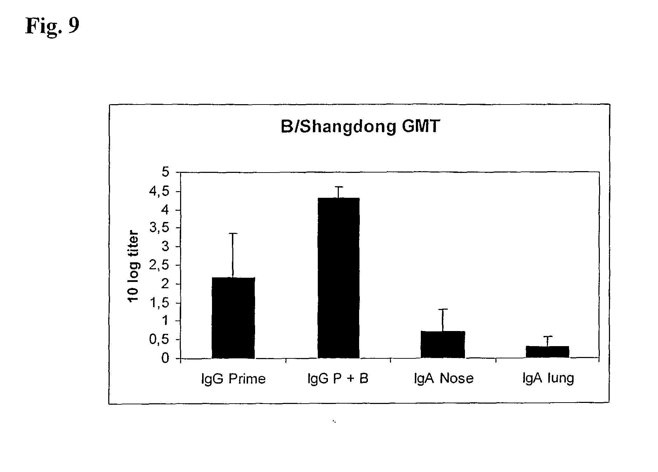

Figure 9 shows the geometric mean titers (GMT) + standard error of the mean for serum IgG after one (Prime) or two (prime and boost: P + B) intranasal immunizations with co-micelles of B/Shangdong antigenic integral membrane proteins in complex with N-palmitoyl-S-2,3(bispalmitoyloxy)-propyl-cysteinyl-seryl-(lysyl)₃-lysine. It also shows the nose and lung IgA titers after two immunizations (interval of two weeks between immunizations) in groups of ten mice as described in example 5. Samples were taken two weeks after the first or three weeks after the second immunization.

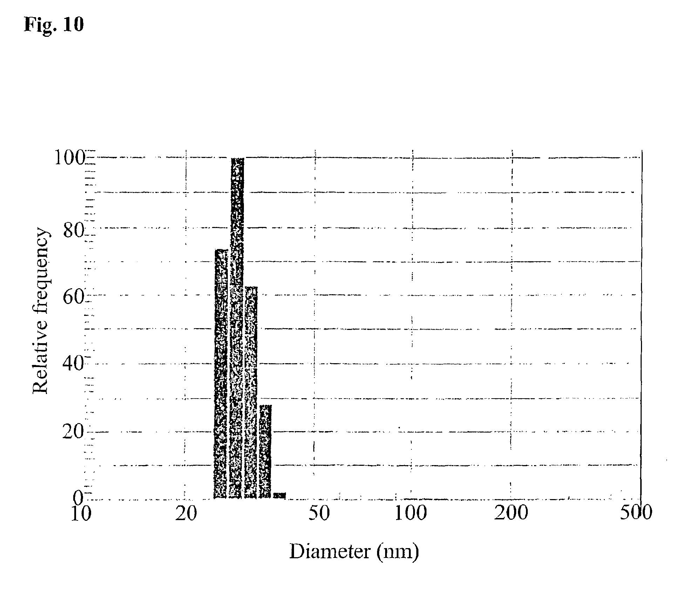

Figure 10 shows the size distribution of samples of co-micelles according to the present invention formed from antigens from influenza A/New Caledonia in complex with N-palmitoyl-S-2,3(bispalmitoyloxy)-propyl-cysteinyl-seryl-(lysyl)₃-lysine, at a 1:1 weight ratio, as analyzed in a Nicomp 380 particle size analyzer (Particle Sizing Systems, Inc. Santa Barbara, CA, USA) using the "solid particle" analysis mode and Nicomp number-weigthed distribution analysis.

### DESCRIPTION OF THE INVENTION

The present invention provides novel means and methods that solve at least part of the problems and difficulties outlined above. The adjuvant activity of certain amphiphilic compounds, such as (synthetic) lipopeptides is known in the art (Lex et al. 1986; Schlecht et al. 1989; Reitermann et al. 1989; Baier et al. 2000; Huber et al. 2002; Erdile and Guy 1997). Lipopeptides are useful mucosal adjuvants, but have found little practical applicability due to their low water-solubility. Although versions, such as *N-*palmitoyl-S-2,3(bispalmitoyloxy)-propyl-cysteinyl-seryl-(lysyl)₃-lysine, have been synthesized with improved water solubility, even these require suspension by ultra-sonication just prior to mixing with the antigen and application to an animal (Manufacturer's instructions, EMC Microcollections GmbH). Therefore, as such these lipopeptides are cannot be formulated as part of a commercial vaccine. Integral membrane proteins of pathogens such as viruses are also difficult antigens, because they are insoluble in water. Suspensions formed from such proteins ("subunit" vaccines) containing random aggregates of proteins are used in injected vaccines, but the presentation of these protein to the immune system is so inefficient, that they have no activity at all when applied intranasally. The inventor of the present invention has now provided methods and means for making novel compositions referred to as co-micelles that comprise such proteins in a complex with amphiphilic adjuvants such as these lipopeptides and therefore has provided new products useful in vaccines against different kinds of disorders and infectious disease, that can be applied via intranasal and/or oral delivery. The invention relates to a co-micelle comprising an amphiphilic compound and an antigen, wherein said amphiphilic compound and said antigen interact through hydrophobic interactions, wherein said amphiphilic compound has adjuvant activity, wherein said antigen is an amphiphilic protein or a fragment thereof, and wherein the hydrophobic parts of said amphiphilic compound and said antigen are directed towards the inside of said co-micelle in an aqueous environment.

The invention further relates to a method for producing a co-micelle, comprising the steps of: i) contacting an amphiphilic compound having adjuvant activity and an antigen solubilized in a solution comprising a detergent; and ii) decreasing the detergent concentration under conditions that cause (or allow) the formation of co-micelles in which said amphiphilic compound and said antigen interact through hydrophobic interactions, wherein said antigen is an amphiphilic protein or a fragment thereof. The invention also relates to co-micelles obtainable by the methods of the invention.

Moreover, the invention provides pharmaceutical preparations comprising co-micelles according to the invention, and a therapeutically acceptable carrier, as well as the use of co-micelles or pharmaceutical preparations according to the invention in therapy, prophylaxis or diagnosis, either by intranasal or oral delivery.

### DETAILED DESCRIPTION OF THE INVENTION

The present invention relates to methods for producing a co-micelle, said method comprising the steps of: contacting an amphiphilic compound having adjuvant activity and an antigen in a solution comprising a detergent, wherein said antigen is an amphiphilic protein or a fragment thereof; and decreasing the detergent concentration under conditions that cause the formation of co-micelles, in which said amphiphilic compound and said antigen interact through hydrophobic interactions. Preferably, said antigen is an (amphiphilic) membrane protein. Also preferred are surface proteins of infectious agents, such as antigenic surface proteins from viruses. Also preferred is a method according to the invention wherein said antigen is an amphiphilic outer membrane protein. Highly preferred are methods according to the invention wherein said antigen comprises an integral membrane protein. In one embodiment, the produced co-micelles are harvested from the solution in a subsequent step. In another embodiment, said antigen is purified before contacting said amphiphilic compound having adjuvant activity. In yet another embodiment, said antigen is de-lipidated (made substantially free of lipids from the membrane the antigen previously interacted with) before contacting said amphiphilic compound having adjuvant activity. De-lipidation is performed to prevent the formation of lipid bilayer membranes. It is to be understood that delipidation is never 100%.

The invention also relates to products obtainable by the methods of the present invention. The invention provides co-micelles comprising an amphiphilic compound having adjuvant activity, and an antigen, wherein said amphiphilic compound and said antigen interact through hydrophobic interactions, wherein said antigen is at least one amphiphilic protein or a fragment thereof and wherein the hydrophobic parts of said amphiphilic compound and said antigen are directed towards the inside of said co-micelle in an aqueous environment. Preferably, said antigen present in the co-micelle according to the invention, is an (amphiphilic) membrane protein. Also preferred proteins are surface proteins of infectious agents, such as antigenic membrane proteins of viruses. Preferred is also a co-micelle according to the invention wherein said antigen is an amphiphilic outer membrane protein. Highly preferred are co-micelles according to the invention wherein said antigen comprises an integral membrane protein.

'Co-micelles' as used herein refers to micelles formed from at least two different amphiphilic molecules, wherein at least one amphiphilic molecule has adjuvant activity and at least one other amphiphilic molecule (the antigen) is an amphiphilic protein or a fragment thereof. The co-micelle does not comprise a lipid-bilayer as in compositions generally referred to as virosomes. Preferably, the co-micelles have a size of between 25 and 42 nm in diameter. In a highly preferred embodiment said antigen is an integral membrane protein. Integral membrane proteins are generally insoluble in water but can be extracted from a membrane by the action of a detergent or organic solvent, using general methods known to the person of skill in the art. The properties described above distinguish integral membrane proteins from peripheral membrane proteins. Peripheral membrane proteins can be washed from the membrane with brine. Integral membrane proteins are also distinguished from soluble proteins, which are not associated with membranes. Since such integral membrane proteins are very poorly soluble in water, they cannot elicit a strong immune response on their own. The present invention now provides a method for producing novel compositions referred to as co-micelles that comprise such insoluble integral membrane proteins, thereby providing means for producing novel vaccine compositions that are suitable for intranasal and oral delivery.

By 'amphiphilic antigens' as used herein, are meant proteins, fragments thereof, stretches of amino acids, peptides or polypeptides that have at least one hydrophilic and at least one hydrophobic moiety and that can elicit an immune response in the host whereto it is delivered. Non-limiting examples of such antigens, preferably integral membrane proteins, are membrane proteins from tumor cells, from bacteria, parasites, yeasts and the envelope of viruses.

'Integral membrane proteins' as used herein refers to proteins that comprise a hydrophobic domain capable of spanning a lipid bilayer membrane, and do so in the organism in which they are present in nature. It is to be understood that 'integral' does not solely refer to 'full-length'; it also refers to proteins that comprise mutations, deletions, additions, protein-swaps, peptide-swaps, and/or other (post-translational and/or chemically induced) modifications. It is therefore to be understood that 'integral' refers to proteins, or fragments thereof, that comprise amphiphilic parts which are generally the fragments of the protein that comprise the membrane-spanning domain or a part thereof. Mutations in the outer (generally antigenic) part of the protein as well as mutations in the membrane spanning domain are encompassed by the claims in the present disclosure, as long as the protein undergoes hydrophobic interactions with the amphiphilic compounds used in the methods and co-micelles of the present invention.

'Amphiphilic compounds' as used herein refers to compounds that have at least one hydrophobic and at least one hydrophilic moiety and are therefore neither completely soluble in water nor in organic solvents.

"Amphiphilic compounds with adjuvant activity" as used herein, are meant naturally occurring or (partly) synthetic compounds that have at least one hydrophilic and at least one hydrophobic moiety and that are capable of forming a co-micelle with an antigen of interest in an aqueous environment under conditions that allow co-micelle formation. Examples of such amphiphilic compounds are lipopeptides. Preferred lipopeptides are lipopeptides that are recognized by Toll-like receptors (TLRs). Toll-like receptors are transmembrane proteins with leucine-rich repeats that carry structural homology to the Toll protein in *Drosophila* and are activated by a variety of microbial signals from bacteria not normally present in the host. All lipopeptides listed in Table 1 are known to interact with TLRs. Other examples of amphiphilic compounds are glycolipids and peptides that target (bind to) receptors on antigen presenting cells such as dendritic cells. Preferably, dendritic cells are targeted by said glycolipids and/or said peptides. In one embodiment of the invention said amphiphilic compound is derivable from a bacterium, while it is also preferred that said amphiphilic compound is acceptable for use in humans.

'Derivable' as used herein means that the amphiphilic compound may be directly derived (obtained and/or purified to a certain extent) from (for instance) a bacterium although it also means that it may be (bio) synthetically, enzymatically produced or otherwise (re) produced. This is not critical to the invention. In a preferred aspect of the invention, said amphiphilic compound comprises a lipopeptide. A lipopeptide consists of a peptide covalently bonded to one or more hydrophobic carbohydrate chains, one or more fatty acids, lipids, ceramides, plasmalogens, alkyl or alkene chains, or sterols. Preferred lipopeptides that can be used for the production of co-micelles according to the invention are listed in Table 1. Highly preferred is the lipopeptide *N-*palmitoyl-S-2,3(bispalmitoyloxy)-propyl-cysteinyl-seryl-(lysyl)3-lysine.

In another embodiment of the invention the amphiphilic compound comprises a glycolipid. Glycolipids are lipids covalently bonded to one or more sugars. Examples are phosphatidyl inositol mannoside, alpha-galactosylceramide or a derivative of either one. As 'derivatives' of alpha-galactosylceramide that are useful in co-micelles according to the present invention are to be understood also all derivatives as disclosed in US patent no. 5,936,076 having adjuvant activity.

In a preferred embodiment, the amphiphilic compound present in the co-micelle is pharmaceutically acceptable for use in humans. The person skilled in the art would recognize an amphiphilic compound as being acceptable for use in humans. Such compounds should be non-toxic or at least relatively well tolerated and eliciting a significant effect in humans. Adjuvants are substances that, in combination with an antigen, stimulate the immune system, thereby provoking, enhancing or facilitating the immune response. Because the amphiphilic compounds of the invention are not covalently linked, processing of the antigen and presentation of its epitopes to the immune system is in essence identical to that of the natural protein alone, ensuring good recognition of the protein present on the natural pathogen.

In another aspect of the present invention, the amphiphilic compound present in the co-micelle according to the invention, comprises a peptide, preferably comprising the sequence of the notch ligand jagged-1 (Weijzen et al. 2002), while in another embodiment, the peptides comprise parts of the *Staphylococcus Aureus* protein A.

An important aspect of the present invention is that the co-micelles of the present invention can be applied for intranasal delivery of antigens that would not normally elicit a sufficient immune response in the treated subject to protect against subsequent infection by the pathogenic organism comprising the antigen. The antigens that are part of the co-micelle according to the invention should have a hydrophobic part that is directed towards the inside of the co-micelle particle. Many pathogenic entities such as viruses, bacteria, yeasts and parasites contain such proteins, for example in their membrane (also called envelope in the case of viruses) or cell wall. Examples of antigens that have hydrophobic elements and that are suited to be part of a co-micelle according to the invention are the integral membrane proteins of enveloped viruses. In preferred embodiments, said antigen is derived from a virus, a parasite or a bacterium. Especially preferred are co-micelles, wherein said antigen is derived from influenza virus. Proteins from influenza virus that can be used in co-micelles of the present invention are preferably the hemagglutinin (HA) protein, the neuraminidase (NA) protein and/or the M2 protein, alone or in combination. A preferred combination comprises HA and NA protein present in a single preparation of co-micelles according to the present invention.

Antigens that can be applied and used in the formation of the co-micelles according to the invention can be derived from all sorts of viruses from different virus families. Non-limiting examples of such families are: Retroviridae, Adenoviridae, Paramyxoviridae, Flaviviridae, Herpesviridae, Bunyaviridae; Hantaviridae, Papovaviridae, Rhabdoviridae, Coronaviridae, Alphaviridae, Arteriviridae, Filoviridae, Arenaviridae and Poxviridae. Non-limiting examples of viruses from these families that can be used for preparing compositions of the present invention are: Human Immunodeficiency virus (HIV), FIV, SIV, rubella virus, parainfluenza virus, several adenovirus serotypes, measles virus, mumps virus, respiratory syncytial virus, human metapneumovirus, yellow fever virus, dengue virus, Hepatitis C Virus (HCV), Japanese encephalitis virus (JEV), tick-borne encephalitis virus, St. Louis encephalitis virus, West Nile virus, Herpes Simplex virus, cytomegalovirus, Epstein-Barr virus, Hanta virus, human Papillomavirus, rabies virus, human coronavirus (including but not limited to a SARS-causing coronavirus), Sindbis virus, Semliki Forest virus, Ebolavirus, smallpox virus and African swine fever virus.

The invention further relates to the use of a co-micelle according to the invention for the preparation of a medicament for the prophylactic treatment of a viral-induced disease, such as flu.

Although vaccination is generally applied for the prophylactic protection against pathogens or for the treatment of diseases following pathogenic infection, the person skilled in the art is also aware of the application of some vaccines for tumor-treatment. Moreover, an increasing number of tumor-specific proteins are found to be proper entities that can be targeted by human or humanized antibodies. Such tumor-specific proteins are also within the scope of the present invention. Many tumor specific antigens are known in the art. Therefore, in one preferred embodiment, the present invention provides co-micelles comprising a tumor-specific antigen. The invention therefore also relates to the use of a co-micelle according to the invention for the preparation of a medicament for the treatment of a tumor-related disease, such as cancer.

In one aspect, the present invention provides a method for producing a co-micelle, comprising the steps of: contacting an amphiphilic compound having adjuvant activity and an antigen in a solution comprising a detergent; and decreasing the detergent concentration under conditions that allow (or cause) the formation of co-micelles, wherein said amphiphilic compound and said antigen, present in said co-micelle, interact through hydrophobic interactions. Preferably, the method for producing a co-micelle disclosed by the present invention comprises the step of purifying said co-micelle. Adjuvants are substances that, in combination with an antigen, stimulate the immune system, thereby provoking, enhancing or facilitating the immune response. Detergents are amphiphilic molecules with surface activity.

The present invention provides a pharmaceutical preparation comprising a co-micelle according to the invention, and a therapeutically acceptable carrier. Therapeutically acceptable carriers for intransal delivery are substances that not toxic, or at least known to be tolerated, when applied to the nose. Therapeutically acceptable carriers for intranasal delivery are for instance water, buffered saline solutions, glycerin, polysorbate 20, cremophor EL, inulin, cyclodextrins, ethanolamine, glycine, and an aqueous mixture of caprylic/capric glyceride. Since a number of known medicaments in the art are applied intranasally, it is known to the person skilled in the art how such intranasal applications take place, for instance via injection or sprays.

Furthermore, the present invention relates to the use of a co-micelle according to the invention, or a pharmaceutical preparation according to the invention, in therapy, prophylaxis or diagnosis, and/or for the use of a co-micelle according to the invention for the preparation of a medicament for the treatment and/or the prevention of a disease caused by an infectious agent, such as influenza virus or any virus as mentioned herein. Preferably the pharmaceutical preparation according to the invention is used for intranasal delivery. In another embodiment the pharmaceutical preparation according to the invention may be used for oral or parenteral delivery.

The present invention provides antigen-presenting complexes, herein referred to as co-micelles, that, by definition, do not contain a lipid bilayer, and that preferably comprise substantially de-lipidated integral membrane proteins as antigenic determinant mixed with amphiphilic compounds that have adjuvant activity. Such integral membrane proteins are not by themselves soluble in water, and have to be extracted from membranes using a detergent or organic solvent. Important but not limiting examples of the amphiphilic compounds are glycolipids, lipopeptides and (amphiphilic) peptides. The amphiphilic compounds that are used in the co-micelles of the present invention should be therapeutically acceptable for use in humans, in contrast to for instance Quil A^{®} or saponins that are amphiphils that have been tested in certain settings in the art. The proteins in the co-micelles of the present invention are oriented in the same way as they appear on the viral or cellular membrane, but may present epitopes that are normally partially or at least temporarily hidden when present in a membrane lipid bilayer. Stimulation of the immune system by these antigen-presenting complexes may be due to a combination of their specific recognition by cells-of the immune system, their particular character, the presentation of the protein, and the uncovering of hidden epitopes.

### EXAMPLES

### Example 1. Production of a lipopeptide-influenza membrane protein co-micelle.

Influenza virus was produced by growing virus acquired from the World Influenza Center in embryonated eggs, purified by differential ultra-centrifugation, and inactivated by formaldehyde treatment according to established standard procedures known by persons skilled in the art.

The purified and concentrated virus was incubated with a suitable detergent, such as beta-D-octylglucoside (Boehringer, Mannheim, Germany) at a concentration of 60 mM (a concentration above the detergent's critical micelle concentration is required), for 30 min at 4°C, in an isotonic buffer at neutral pH: 145 mM NaCl, 2.5 mM HEPES, pH 7.4 (Buffer A). The viral nucleocapsid and matrix protein were then removed by centrifugation at 100,000xg for 40 min at 4°C. The pellet was discarded, and the supernatant was used for purification of viral glycoproteins by passing the supernatant over an affinity chromatography column containing *Ricinis communis* lectin coupled to Sepharose beads (Sigma). The column was washed with 5 volumes of buffer A containing 30 mM beta-D-octylglucoside to remove the viral lipids. HA was eluted with buffer A containing 0.2 M D(+)galactose and 30 mM beta-D-octylglucoside, followed by pooling the fractions containing HA.

Using this procedure, no proteins other than HA were detected on gel (Figure 2), indicating that HA was substantially pure. No detectable lipids remained, as determined by the Böttcher procedure, with nanomolar sensitivity (Böttcher et al. 1961). N-palmitoyl-S-2,3(bispalmitoyloxy)-propyl-cysteinyl-seryl-(lysyl)₃-lysine was suspended in buffer A containing 30 mM beta-D-octylglucoside, and mixed, at a weight ratio of lipopeptide to protein of 1:1, with the solution containing the membrane glycoprotein (for instance HA from influenza), that was purified as described above, and then the detergent was removed by extensive dialysis. Other useful lipopeptides, next to the N-palmitoyl-S-2,3(bispalmitoyloxy)-propyl-cysteinyl-seryl-(lysyl)₃-lysine applied above, and that can be used for the production of co-micelles according to the invention, are presented in Table 1.

Dialysis or ultra-filtration was carried out using membranes with a cut-off of 30 kD in buffer A, using methods known to the skilled person. The rate of detergent removal was carefully controlled to prevent aggregation and removal of detergent by dialysis was done against 1000 volumes of buffer A for 24 h at 4°C, with 3 changes of buffer.

### Example 2. Purification and de-lipidation of the influenza membrane glycoproteins on a DEAE column.

Formaldehyde-inactivated influenza virus was produced as in example 1. The purified virus was concentrated by ultracentrifugation, and the viral pellet was resuspended with beta-D-octylglucoside (Boehringer, Mannheim, Germany) at a concentration of 60 mM for 30 min at 4°C in a low-salt buffer: 10 mM TRIS, 1 mM EDTA, pH 7.4 (Buffer A). The viral nucleocapsid and matrix protein were then removed by centrifugation at 100,000xg for 35 min at 4°C. The pellet was discarded, and the supernatant used for purification of viral glycoproteins.

The viral glycoproteins, mainly hemagglutinin (HA) and neuraminadase (NA), present in the supernatant containing the solubilized viral membrane, were then de-lipidated by application of the sample to a DEAE cellulose ion exchange column (Servacel, DEAE-GS, analytical grade cellulose ion exchanger: 1 ml packed column volume was used per 0.3 mg of viral membrane protein), equilibrated in buffer A, and the column washed with 10 column volumes of buffer A.

The following steps are dependent on the virus strain: for the (formaldehyde-inactivated) A/Panama strain, this wash was followed by 5 volumes of buffer B: 0.1 M NaCl, 10 mM TRIS, 1 mM EDTA, 30 mM octylglucoside, pH 7.4, resulting in the removal of the 96% of viral membrane lipids. The viral membrane proteins were harvested by elution with 5 volumes of buffer C: 1 M NaCl, 10 mM TRIS, 1 mM EDTA, 30 mM octylglucoside, pH 7.4.

For the (formaldehyde-inactivated) A/New Caledonia strain, the wash with buffer A was followed by 5 volumes of buffer D: 0.05 M NaCl, 10 mM TRIS, 1 mM EDTA, 30 mM octylglucoside, pH 7.4, resulting in the removal of the 98% of viral membrane lipids (Figure 3). The viral membrane proteins were harvested by elution with 5 volumes of buffer C: 1 M NaCl, 10 mM TRIS, 1 mM EDTA, 30 mM octylglucoside, pH 7.4 (Figure 3).

For the (formaldehyde-inactivated) B/Shangdong strain, after the wash with buffer A, the proteins were eluted by adding 5 volumes of buffer E: 0.05 M NaCl, 10 mM TRIS, 1 mM EDTA, 30 mM octylglucoside, pH 7.4, resulting in the removal of the 96 % of viral membrane lipids.

### Example 3. Production of N-palmitoyl-S-2,3(bispalmitoyloxy)-propyl-cysteinyl-seryl- (lysyl) 3-lysine influenza A/New Caledonia 20/99 membrane protein co-micelles, and physicochemical characterization of co-micelle formation.

The following steps are independent of the virus strain used. N-palmitoyl-S-2,3(bispalmitoyloxy)-propyl-cysteinyl-seryl-(lysyl)₃-lysine, suspended in buffer A, containing 60 mM beta-D-octylglucoside, was added to the membrane proteins, purified as in example 2, typically at a ratio of 1 mg of proteins per mg of lipopeptide. Subsequently, the detergent was removed by extensive dialysis or ultrafiltration against a buffer isotonic with human physiological conditions at neutral pH: 145 mM NaCl, 2.5 mM HEPES, pH 7.4 (buffer F). Dialysis was carried out using a Slide-a-Lyzer (Pierce). The rate of detergent removal was carefully controlled to prevent aggregation, which could be followed by visual inspection, and typically required dialysis at a ratio of less than 1000 volumes of buffer F until the onset of co-micelle formation. After that, removal of residual detergent required dialysis against at least 1000 volumes of buffer F for at least 24 h at 4°C. Formation of co-micelles was monitored by spectrometry; at the onset of co-micelle formation, there was a sudden increase in absorbance at 450 nm.

The presence of co-micelles after dialysis was demonstrated by negative stain electron microscopy, using methods known in the art; the complexes were 20-40 nm in diameter, and show multiple hemagglutinin spikes around a central core, which was most likely formed by the lipopeptides (Figure 4). Micelles formed by dialysis of purified proteins alone, in the absence of lipopeptide, showed spikes, but lacked the central core.

The physical association of the proteins and lipopeptide in co-micelles was demonstrated by equilibrium density gradient ultracentrifugation on continuous 10-40% sucrose gradients in buffer D. At a 1:1 weight ratio of protein to lipopeptide, a peak containing 50% of the protein and all of the lipopeptide, with a density of 1.122-1.135 g/ml was observed (Figure 5). More protein remained unincorporated at higher protein to lipopeptide ratios, while the density of the co-micelles varies with the protein to lipopeptide ratio (Figure 6): at a 14:1 weight ratio of protein to lipopeptide, the density of the particles was 1.197 g/ml (Figure 6 panel A), at a 3:1 ratio there was a broad peak between 1.181 and 1.197 g/ml (Panel B), and at 1:2 ratio it was 1.122 g/ml (Panel C). The presence of the lipopeptide was measured by two-dimensional thin-layer chromatography of chloroform extracts of fractions from the gradient, prepared according to Folch (1957); the silica gel TLC plates were first developed in chloroform/methanol/water 65/25/4 and subsequently in N-butanol/acetic acid/water 2/1/1; lipopeptides were stained by spraying with a butanolic suspension of ninhydrin 0.1 % W/V, developing at 60°C, and quantitated by measuring the intensity of the purple color that was developed (Figure 5). In all cases, the lipopeptides were present in the fractions that contained the bulk of the protein, demonstrating the physical association of lipopeptides and protein and corroborating the results of the electron microscopy.

### Example 4. Intranasal immunization experiments using antigen presenting complexes prepared from A/Panama membrane proteins and N-palmitoyl-S-2,3(bispalmitoyloxy)-propyl-cysteinyl-seryl-(lysyl)3-lysine.

Intranasal immunization with an antigen presenting complex containing the influenza virus A/Panama hemagglutinin and N-palmitoyl-S-2,3(bispalmitoyloxy)-propyl-cysteinyl-seryl-(lysyl)₃-lysine, at a 1:1 weight ratio of lipopeptide to protein was prepared as described above, and was compared to intranasal instillation of buffer only, micelles containing hemagglutinin but not the lipopeptide, a standard subunit vaccine, and to virosomes. To prepare micelles without the lipopeptide, a viral membrane protein supernatant was prepared as in example 2, and then the detergent was removed by dialysis against buffer A, yielding a preparation of HA micelles that is commonly refered to as "HA rosettes" in literature. Virosomes were prepared form A/Panama membranes, as described in Stegmann et al. (1987). Six to eight-week old female Balb/C mice were immunized with a total of 5 µg per mouse by intranasal instillation of 5 µl of antigen into each nostril under isoflurane/NO₂ anesthesia, and kept on their back, anaesthesized, for 3-5 min thereafter. In most groups, a booster instillation was given two weeks after the first application. Blood, nasal- and lung washes were collected, either two weeks after primary immunization, or three weeks after the second. Lung washes were performed by injection of 1 ml of phosphate buffered saline (PBS) into the lungs via a syringe connected to the trachea, followed by aspiration of the fluid. Nasal washes were collected by aspirating 0.5 ml of PBS retrograde, via the trachea, into the nasopharynx, the lavage fluid being subsequently collected at the nostrils. Protease inhibitor mix (Boehringer) was added to the washes immediately, they were placed on ice, while debris and cellular components were immediately removed from the lavages by centrifugation, and all samples were processed within 24 hrs. Blood samples were collected retroorbitally or from the vena cava. Antigen-specific IgA and IgG were measured by ELISA. For this purpose, ELISA plates (Greiner Bio-one) were coated with commercial influenza subunit vaccine (Solvay, 100 µl per well, at a concentration of 0.2 ng per well, overnight at 37°C) in coating buffer (0.05 M sodium bicarbonate, pH 9.6), washed once with coating buffer, blocked for 45 min at 37°C with 100 µl blocking buffer (2.5% milk powder in coating buffer), and washed once with coating buffer and twice with PBS/0.05% w/v Tween-20.

Serial twofold dilutions of samples (100 µl per well) were then applied to the plates, and incubated for 90 min at 37°C, the plates were washed with PBS/Tween 3 times, incubated with a 1:5000 dilution of the appropriate goat-anti mouse Ig coupled to horse radish peroxidase for 60 min at 37°C, washed three times with PBS/Tween and once with PBS, after which 100 µl staining solution (containing 20 mg o-phenylenediamine, added from a 2 ml ethanolic solution to 100 ml of 50 mM sodium phosphate buffer at pH 5.6, and 20 µl of H₂O₂) was added for 30 min at room temperature. The reactions were stopped by adding 50 µl of H₂SO₄, 50 µl per well, the absorbance at 492 nm was determined in an ELISA reader, and the titers were calculated as the reciprocal dilutions of sample at which the absorbance reads 0.2 OD units above background.

The IgA titers in nose and lung are presented in figure 7, while the serum IgG titers are shown in Figure 8, which clearly show a significant increase of IgA and IgG titers after using the co-micelles of the present invention as compared to HA micelles without lipopeptide or when using the standard subunit vaccine or when applying virosomes. These results strongly indicate that the co-micelles of the present invention are useful for intranasal applications and elicit a strong immune response in the host.

### Example 5. Intranasal immunization experiments using antigen presenting complexes prepared from B/Shangdong membrane proteins and N-palmitoyl-S-2,3(bispalmitoyloxy)-propyl-cysteinyl-seryl-(lysyl)₃-lysine.

Intranasal immunization with antigen presenting complexes containing the influenza virus B/Shangdong hemagglutinin antigen as the integral membrane protein and N-palmitoyl-S-2,3(bispalmitoyloxy)-propyl-cysteinyl-seryl-(lysyl)₃-lysine, at a 1:1 weight ratio of lipopeptide to protein, prepared and characterised as described in examples 2 and 3, was performed according to the protocol of example 4. 6-8 week old female Balb/C mice were immunized with a total of 5 µg per mouse by intranasal instillation of 5 µl of antigen into each nare under isoflurane/NO₂ anesthesia, and kept on their back, anaesthesized, for 3-5 min thereafter. A booster instillation was given two weeks after the first application. Blood was collected at the time of the boost. All blood samples, nasal- and lung washes were collected as described in example 4. Antigen-specific IgA and IgG were measured by ELISA as described in example 4. Antibody titers are presented in Figure 9, clearly indicating that the co-micelle composition as used herein was able to elicit a strong IgG and IgA response upon administration, while a prime/boost set-up significantly elevated the IgG titers.

### Example 6. Size analysis of co-micelles of N-palmitoyl-S-2,3(bispalmitoyloay)-propyl-cysteinyl-seryl-(lysyl)₃-lysine and influenza A/New Caledonia 20/99 membrane protein.

The size of the co-micelles, produced as decribed in example 3 was analyzed in a Nicomp 380 particle size analyzer (Particle Sizing Systems, Inc. Santa Barbara, CA, USA) using the "solid particle" analysis mode and Nicomp number-weigthed distribution analysis known to persons skilled in the art. The particle size distribution is shown in Figure 10. 99.5% of the co-micelles were found to be between 25 and 42 nm in diameter, and 0.4 % had a size of around 110 nm. The average size of the co-micelles was 29.1 nm.

**Table 1. Lipopeptides particularly suitable for making co-micelles according to the invention.**

| |
|---|
| N-palmitoyl-S-2,3(bispalmitoyloxy)-propyl-cysteinyl-seryl-serine |
| S-2,3(bispalmitoyloxy)-propyl-cysteinyl-seryl-serine |
| N-palmitoyl-S-2,3(bispalmitoyloxy)-propyl-cysteinyl-seryl-(prolyl)₃-proline amide |
| N-palmitoyl-S-2,3(bispalmitoyloxy)-propyl-cysteinyl-seryl-(histidyl)₃-histidine |
| N-palmitoyl-S-2,3(bispalmitoyloxy)-propyl-cysteinyl-seryl-(glutamyl)₃-glutaminic acid |
| N-palmitoyl-S-2,3(bispalmitoyloxy)-propyl-cysteinyl-seryl-(lysyl)₃-lysine |
| N-palmitoyl-S-2,3(bispalmitoyloxy)-propyl-cysteine |
| S-2,3(bispalmitoyloxy)-propyl-cysteinyl-seryl-(lysyl)₃ -lysine |
| N-palmitoyl-S-2,3(bisoleoyloxy)-propyl-cysteinyl-seryl-(lysyl)₃-lysine |
| S-2,3(bisoleoyloxy)-propyl-cysteinyl-seryl-(lysyl)₃ -lysine |
| N-palmitoyl-S-2,3(bismyristoyloxy)-propyl-cysteinyl-seryl-(lysyl)₃-lysine |
| S-2,3(bismyristoyloxy)-propyl-cysteinyl-seryl-(lysyl)₃-lysine |
| N-palmitoyl-S-3(palmitoyloxy)-propyl-cysteinyl-seryl-(lysyl)₃-lysine |
| N-palmitoyl-S-2,3 hydroxy-propyl-cysteinyl-seryl-(lysyl)₃-lysine |

### REFERENCES

Baier W, Masihi N, Huber M, Hoffmann P, Bessler WG (2000) Immunobiology 201:391-405
Böttcher CJF, Van Gent CM, Fries CJ (1961) Anal Chim Acta 24:203-204
Cox JC, Sjolander A, Barr IG (1998) Adv Drug Delivery 32:247-271
Erdile LF and Guy B (1997) Vaccine 15:988-996
Gluck et al., (1994) J Infect Dis 181:1129-1132
Huber M, Baier W, Bessler WG, Heinevetter L (2002) Immunobiology 205:61-73
Janeway et al. (2001) Immunobiology, 5th edition, Garland Publishing, New York
Lex et al. (1986) J Immunology 137:2676-2681
Morein et al. (1984) Nature 308:457-460
Ogra PL, Faden H, Welliver RC (2001) Clin Microbiol Rev 14:430-445
Reitermann A, Metzger J, Wiesmuller K-H, Jung G, Bessler WG (1989) Biol Chem 370:343-352
Schlecht S, Wiesmuller K-H, Jung G, Bessler WG (1989) Zbl. Bakt. 271:493-500
Stegmann T, Morselt HWM, Booy FP, Van Breemen JFL, Scherphof G, Wilschut J (1987) EMBO J 6:2651-2659
Weijzen S, Velders MP, Elmishad AG, Bacon PE, Panella JR, Nickoloff BJ, Miele L, Kast WM (2002) J Immunol 169:4237-4238

## Claims

1. A method for producing a co-micelle, comprising the steps of:
i) contacting an amphiphilic compound having adjuvant activity and an antigen in a solution comprising a detergent, wherein said antigen is an amphiphilic protein or fragment thereof; and
ii) decreasing the detergent concentration under conditions that cause the formation of a co-micelle in which said amphiphilic compound and said antigen interact through hydrophobic interactions.

2. A method according to claim 1, wherein said amphiphilic protein is a surface protein of an infectious agent.

3. A method according to claim 1 or 2, wherein said amphiphilic protein is a membrane protein.

4. A method according to any one of claims 1-3, wherein said amphiphilic protein is an outer membrane protein.

5. A method according to any one of claims 1-3, wherein said amphiphilic protein is an integral membrane protein.

6. A method according to any one of claims 1-5, further comprising the step of harvesting said co-micelle from the solution.

7. A method according to any one of claims 1-6, wherein said antigen is purified before contacting said amphiphilic compound.

8. A method according to any one of claims 1-7, wherein said antigen is de-lipidated before contacting said amphiphilic compound.

9. A co-micelle comprising an amphiphilic compound having adjuvant activity and an antigen, wherein said amphiphilic compound and said antigen interact through hydrophobic interactions, wherein said antigen is at least one amphiphilic protein or a fragment thereof, and wherein the hydrophobic parts of said amphiphilic compound and said antigen are directed towards the inside of said co-micelle in an aqueous environment.

10. A co-micelle according to claim 9, wherein said amphiphilic protein is a surface protein of an infectious agent.

11. A co-micelle according to claim 9, wherein said amphiphilic protein is a membrane protein.

12. A co-micelle according to any of claims 9-11, wherein said amphiphilic protein is an outer membrane protein.

13. A co-micelle according to any of claims 9-11, wherein said amphiphilic protein is an integral membrane protein.

14. A co-micelle according to any one of claims 9-13, wherein said amphiphilic compound is pharmaceutically acceptable for use in humans.

15. A co-micelle according to any one of claims 9-14, wherein said amphiphilic compound is derivable from a bacterium.

16. A co-micelle according to any one of claims 9-15, wherein said amphiphilic compound comprises a lipopeptide.

17. A co-micelle according to claim 16, wherein said lipopeptide is selected from the group consisting of: *N-*palmitoyl-S-2,3 (bispalmitoyloxy)-propyl-cysteinyl-seryl-serine, S-2,3 (bispalmitoyloxy)-propyl-cysteinyl-seryl-serine, *N-*palmitoyl-S-2,3 (bispalmitoyloxy)-propyl-cysteinyl-seryl-(lysyl)₃-lysine, S-2,3 (bispalmitoyloxy)-propyl-cysteinyl-seryl-(lysyl)s-lysine, *N-*palmitoyl-S-2,3 (bisoleoyloxy)-propyl-cysteinyl-seryl-(lysyl)s-lysine, S-2,3 (bisoleoyloxy)-propyl-cysteinyl-seryl-(lysyl)₃-lysine *N-*palmitoyl-S-2,3 (bismyristoyloxy)-propyl-cysteinyl-seryl-(lysyl)₃-lysine, S-2,3 (bismyristoyloxy)-propyl-cysteinyl-seryl-(lysyl)s-lysine, *N-*palmitoyl-S-2,3 (palmitoyloxy)-propyl-cysteinyl-seryl-(lysyl)₃-lysine and S-2,3-hydroxy-propyl-cysteinyl-seryl-(lysyl)₃-lysine.

18. A co-micelle according to claim 16, wherein said lipopeptide is *N-*palmitoyl-S-2,3 (bispalmitoyloxy)-propyl-cysteinyl-seryl-(lysyl)₃-lysine.

19. A co-micelle according to claim 16, wherein said lipopeptide is recognized by a Toll-like receptor.

20. A co-micelle according to any of claims 9-15, wherein said amphiphilic compound comprises a glycolipid.

21. A co-micelle according to claim 20, wherein said glycolipid comprises phosphatidyl inositol mannoside and/or alpha-galactosylceramide or a derivative thereof.

22. A co-micelle according to any of claims 9-15, wherein said amphiphilic compound comprises a peptide.

23. A co-micelle according to any one of claims 20-22, wherein said glycolipid or said peptide binds to a receptor on an antigen presenting cell, preferably a dendritic cell.

24. A co-micelle according to claim 10, wherein said infectious agent is a virus.

25. A co-micelle according to claim 24, wherein said virus is influenza virus.

26. A co-micelle according to claim 25, wherein said antigen comprises influenza hemagglutinin (HA) protein and/or neuraminidase (NA) protein and/or the M2 protein.

27. A co-micelle according to claim 24, wherein said antigen is derived from a virus belonging to a family selected from the group consisting of: Retroviridae, Adenoviridae, Paramyxoviridae, Flaviviridae, Herpesviridae, Bunyaviridae; Hantaviridae, Papovaviridae, Rhabdoviridae, Coronaviridae, Alphaviridae, Arteriviridae, Filoviridae, Arenaviridae and Poxviridae.

28. A co-micelle according to claim 9, wherein said antigen is derived from a parasite or a bacterium.

29. A co-micelle according to claim 9, wherein said antigen comprises a tumor-specific antigen.

30. A pharmaceutical preparation comprising a co-micelle according to any one of claims 9-29, and a therapeutically acceptable carrier.

31. A co-micelle according to any one of claims 9-29 for use in therapy, prophylaxis or diagnosis.

32. A pharmaceutical preparation according to claim 30 for use in intranasal delivery.

33. A pharmaceutical preparation according to claim 30 for use in oral delivery.

34. A co-micelle according to any one of claims 9-29, wherein said co-micelle has a size of between 25 and 42 nm in diameter.

## Patentansprüche

1. Verfahren zur Herstellung einer Co-Mizelle, mit den folgenden Schritten:
i) Inkontaktbringen einer amphiphilen Verbindung mit Adjuvansaktivität und eines Antigens in einer ein Detergens enthaltenden Lösung, wobei es sich bei dem Antigen um ein amphiphiles Protein oder Fragment davon handelt; und
ii) Verringern der Detergenskonzentration unter Bedingungen, die zur Ausbildung einer Co-Mizelle führen, in der die amphiphile Verbindung und das Antigen über hydrophobe Wechselwirkungen interagieren.

2. Verfahren nach Anspruch 1, wobei es sich bei dem amphiphilen Protein um ein Oberflächenprotein eines infektiösen Agens handelt.

3. Verfahren nach Anspruch 1 oder 2, wobei es sich bei dem amphiphilen Protein um ein Membranprotein handelt.

4. Verfahren nach einem der Ansprüche 1-3, wobei es sich bei dem amphiphilen Protein um ein Protein der äußeren Membran handelt.

5. Verfahren nach einem der Ansprüche 1-3, wobei es sich bei dem amphiphilen Protein um ein integrales Membranprotein handelt.

6. Verfahren nach einem der Ansprüche 1-5, bei dem man in einem weiteren Schritt die Co-Mizelle aus der Lösung erntet.

7. Verfahren nach einem der Ansprüche 1-6, wobei das Antigen vor dem Inkontaktkommen mit der amphiphilen Verbindung aufgereinigt wird.

8. Verfahren nach einem der Ansprüche 1-7, wobei das Antigen vor dem Inkontaktkommen mit der amphiphilen Verbindung delipidiert wird.

9. Co-Mizelle, umfassend eine amphiphile Verbindung mit Adjuvansaktivität und ein Antigen, wobei die amphiphile Verbindung und das Antigen über hydrophobe Wechselwirkungen interagieren, wobei es sich bei dem Antigen um wenigstens ein amphiphiles Protein oder Fragment davon handelt und wobei die hydrophoben Teile der amphiphilen Verbindung und des Antigens auf das Innere der Co-Mizelle in einer wässrigen Umgebung gerichtet sind.

10. Co-Mizelle nach Anspruch 9, wobei es sich bei dem amphiphilen Protein um ein Oberflächenprotein eines infektiösen Agens handelt.

11. Co-Mizelle nach Anspruch 9, wobei es sich bei dem amphiphilen Protein um ein Membranprotein handelt.

12. Co-Mizelle nach einem der Ansprüche 9-11, wobei es sich bei dem amphiphilen Protein um ein Protein der äußeren Membran handelt.

13. Co-Mizelle nach einem der Ansprüche 9-11, wobei es sich bei dem amphiphilen Protein um ein integrales Membranprotein handelt.

14. Co-Mizelle nach einem der Ansprüche 9-13, wobei die amphiphile Verbindung pharmazeutisch unbedenklich für die Verwendung beim Menschen ist.

15. Co-Mizelle nach einem der Ansprüche 9-14, wobei die amphiphile Verbindung aus einem Bakterium isolierbar ist.

16. Co-Mizelle nach einem der Ansprüche 9-15, wobei die amphiphile Verbindung ein Lipopeptid umfasst.

17. Co-Mizelle nach Anspruch 16, wobei das Lipopeptid ausgewählt ist aus der Gruppe: *N-*Palmitoyl-S-2,3-(bispalmitoyloxy)-propyl-cysteinyl-seryl-serin, S-2,3-(Bispalmitoyloxy)-propyl-cysteinyl-seryl-serin, *N-*Palmitoyl-S-2,3-(bispalmitoyloxy)-propyl-cystein-yl-seryl-(lysyl)₃-lysin, S-2,3-(Bispalmitoyloxy)-propyl-cysteinyl-seryl-(lysyl)₃-lysin, *N-*Palmitoyl-S-2,3-(bisoleoyloxy)-propyl-cysteinyl-seryl-(lys-yl)₃-lysin, S-2,3-(Bisoleoyloxy)-propyl-cysteinyl-sery3-(lysyl)₃-lysin, *N-*Palmitoyl-S-2,3-(bismyristo-yloxy)-propyl-cysteinyl-seryl-(lysyl)₃-lysin, S-2,3-(Bismyristoyloxy)-propyl-cysteinyl-seryl-(lysyl)₃-lysin, *N-*Palmitoyl-S-2,3-(palmitoyloxy)-propyl-cysteinyl-seryl-(lysyl)₃-lysin und S-2,3-Hydroxypropyl-cysteinyl-seryl-(lysyl)₃-lysin.

18. Co-Mizelle nach Anspruch 16, wobei es sich bei dem Lipopeptid um *N-*Palmitoyl-S-2,3-(bispalmitoyloxy)-propyl-cysteinyl-seryl-(lysyl)₃-lysin handelt.

19. Co-Mizelle nach Anspruch 16, wobei das Lipopeptid von einem Toll-ähnlichen Rezeptor erkannt wird.

20. Co-Mizelle nach einem der Ansprüche 9-15, wobei die amphiphile Verbindung ein Glykolipid umfasst.

21. Co-Mizelle nach Anspruch 20, wobei das Glykolipid Phosphatidylinositolmannosid und/oder Alpha-Galactosylceramid oder ein Derivat davon umfasst.

22. Co-Mizelle nach einem der Ansprüche 9-15, wobei die amphiphile Verbindung ein Peptid umfasst.

23. Co-Mizelle nach einem der Ansprüche 20-22, wobei das Glykolipid oder das Peptid an einen Rezeptor auf einer Antigen präsentierenden Zelle, vorzugsweise einer dendritischen Zelle, bindet.

24. Co-Mizelle nach Anspruch 10, wobei es sich bei dem infektiösen Agens um ein Virus handelt.

25. Co-Mizelle nach Anspruch 24, wobei es sich bei dem Virus um Influenzavirus handelt.

26. Co-Mizelle nach Anspruch 25, wobei das Antigen Influenza-Hämagglutinin-(HA-)Protein und/oder Neuraminidase-(NA-)Protein und/oder das M2-Protein umfasst.

27. Co-Mizelle nach Anspruch 24, wobei das Antigen aus einem Virus isoliert wird, das zu einer aus der folgenden Gruppe ausgewählten Familie gehört: Retroviridae, Adenoviridae, Paramyxoviridae, Flaviviridae, Herpesviridae, Bunyaviridae; Hantaviridae, Papovaviridae, Rhabdoviridae, Coronaviridae, Alphaviridae, Arteriviridae, Filoviridae, Arenaviridae und Poxviridae.

28. Co-Mizelle nach Anspruch 9, wobei das Antigen aus einem Parasiten oder einem Bakterium isoliert wird.

29. Co-Mizelle nach Anspruch 9, wobei das Antigen ein tumorspezifisches Antigen umfasst.

30. Pharmazeutische Zubereitung, umfassend eine Co-Mizelle nach einem der Ansprüche 9-29 sowie einen therapeutisch unbedenklichen Trägerstoff.

31. Co-Mizelle nach einem der Ansprüche 9-29 zur Verwendung in der Therapie, Prophylaxe oder Diagnostik.

32. Pharmazeutische Zubereitung nach Anspruch 30 zur Verwendung bei intranasaler Zuführung.

33. Pharmazeutische Zubereitung nach Anspruch 30 zur Verwendung bei oraler Zuführung.

34. Co-Mizelle nach einem der Ansprüche 9-29, wobei die Co-Mizelle einen Durchmesser zwischen 25 und 42 nm aufweist.

## Revendications

1. Méthode de production d'une co-micelle, comprenant les étapes consistant à :
i) mettre en contact un composé amphiphile ayant une activité d'adjuvant et un antigène dans une solution comprenant un détergent, où ledit antigène est une protéine amphiphile ou un fragment de celle-ci ; et
ii) réduire la concentration en détergent dans des conditions qui provoquent la formation d'une co-micelle où ledit composé amphiphile et ledit antigène interagissent par l'intermédiaire d'interactions hydrophobes.

2. Méthode selon la revendication 1, dans laquelle ladite protéine amphiphile est une protéine de surface d'un agent infectieux.

3. Méthode selon la revendication 1 ou 2, dans laquelle ladite protéine amphiphile est une protéine membranaire.

4. Méthode selon l'une quelconque des revendications 1-3, dans laquelle ladite protéine amphiphile est une protéine membranaire externe.

5. Méthode selon l'une quelconque des revendications 1-3, dans laquelle ladite protéine amphiphile est une protéine membranaire intégrale.

6. Méthode selon l'une quelconque des revendications 1-5, comprenant en outre l'étape consistant à collecter ladite co-micelle à partir de la solution.

7. Méthode selon l'une quelconque des revendications 1-6, dans laquelle ledit antigène est purifié avant la mise en contact avec ledit composé amphiphile.

8. Méthode selon l'une quelconque des revendications 1-7, où ledit antigène est délipidé avant la mise en contact avec ledit composé amphiphile.

9. Co-micelle comprenant un composé amphiphile possédant une activité d'adjuvant et un antigène, où ledit composé amphiphile et ledit antigène interagissent par l'intermédiaire d'interactions hydrophobes, où ledit antigène est au moins une protéine amphiphile ou un fragment de celle-ci, et où les parties hydrophobes dudit composé amphiphile et dudit antigène sont dirigées vers l'intérieur de ladite co-micelle dans un environnement aqueux.

10. Co-micelle selon la revendication 9, dans laquelle ladite protéine amphiphile est une protéine de surface d'un agent infectieux.

11. Co-micelle selon la revendication 9, dans laquelle ladite protéine amphiphile est une protéine membranaire.

12. Co-micelle selon l'une quelconque des revendications 9-11, dans laquelle ladite protéine amphiphile est une protéine membranaire externe.

13. Co-micelle selon l'une quelconque des revendications 9-11, dans laquelle ladite protéine amphiphile est une protéine membranaire intégrale.

14. Co-micelle selon l'une quelconque des revendications 9-13, dans laquelle ledit composé amphiphile est pharmaceutiquement acceptable pour une utilisation chez l'homme.

15. Co-micelle selon l'une quelconque des revendications 9-14, dans laquelle ledit composé amphiphile peut être dérivé d'une bactérie.

16. Co-micelle selon l'une quelconque des revendications 9-15, dans laquelle ledit composé amphiphile comprend un lipopeptide.

17. Co-micelle selon la revendication 16, dans laquelle ledit lipopeptide est choisi dans le groupe constitué par la N-palmitoyl-S-2,3-(bispalmitoyloxy)-propyl-cystéinyl-séryl-sérine, la S-2,3-(bispalmitoyloxy)propyl-cystéinyl-séryl-sérine, la N-palmitoyl-S-2,3-(bispalmitoyloxy)-propyl-cystéinyl-séryl-(lysyl)₃-lysine, la S-2,3-(bispalmitoyloxy)propyl-cystéinyl-séryl-(lysyl)₃-lysine, la N-palmitoyl-S-2,3-(bisoléoyloxy)-propyl-cystéinyl-séryl-(lysyl)₃-lysine, la S-2,3-(bisoléoyloxy)-propyl-cystéinyl-séryl-(lysyl)₃-lysine, la N-palmitoyl-S-2,3-(bismyristoyloxyjpropyl-cystéinyl-séryl-(lysyl)₃-lysine, la S-2,3-(bismyristoyloxy)-propyl-cystéinyl-séryl-(lysyl)₃-lysine, la N-palmitoyl-S-2,3-(palmitoyloxy)propyl-cystéinyl-séryl-(lysyl)₃-lysine et la S-2,3-hydroxy-propyl-cystéinyl-séryl-(lysyl)₃-lysine.

18. Co-micelle selon la revendication 16, dans laquelle ledit lipopeptide est la N-palmitoyl-S-2,3-(bispalmitoyloxy)-propyl-cystéinyl-séryl-(lysyl)₃-lysine.

19. Co-micelle selon la revendication 16, dans laquelle ledit lipopeptide est reconnu par un récepteur de type Toll.

20. Co-micelle selon l'une quelconque des revendications 9-15, dans laquelle ledit composé amphiphile comprend un glycolipide.

21. Co-micelle selon la revendication 20, dans laquelle ledit glycolipide comprend un phosphatidyl inositol mannoside et/ou un alpha-galactosylcéramide ou un dérivé de ceux-ci.

22. Co-micelle selon l'une quelconque des revendications 9-15, dans laquelle ledit composé amphiphile comprend un peptide.

23. Co-micelle selon l'une quelconque des revendications 20-22, dans laquelle ledit glycolipide ou ledit peptide se fixe à un récepteur ou une cellule présentant l'antigène, préférablement une cellule dendritique.

24. Co-micelle selon la revendication 10, dans laquelle ledit agent infectieux est un virus.

25. Co-micelle selon la revendication 24, dans laquelle ledit virus est le virus influenza.

26. Co-micelle selon la revendication 25, dans laquelle ledit antigène comprend la protéine hémagglutinine (HA) de l'influenza et/ou la protéine neuraminidase (NA) et/ou la protéine M2.

27. Co-micelle selon la revendication 24, dans laquelle ledit antigène est dérivé d'un virus appartenant à une famille choisie dans le groupe constitué par : Retroviridae, Adenoviridae, Paramyxoviridae, Flaviviridae, Herpesviridae, Bunyaviridae, Hantaviridae, Papovaviridae, Rhabdoviridae, Coronaviridae, Alphaviridae, Arteriviridae, Filoviridae, Arenaviridae et Poxviridae.

28. Co-micelle selon la revendication 9, dans laquelle ledit antigène est dérivé d'un parasite ou d'une bactérie.

29. Co-micelle selon la revendication 9, dans laquelle ledit antigène comprend un antigène spécifique des tumeurs.

30. Préparation pharmaceutique comprenant une co-micelle selon l'une quelconque des revendications 9-29, et un véhicule thérapeutiquement acceptable.

31. Co-micelle selon l'une quelconque des revendications 9-29, destinée à une utilisation en thérapie, prophylaxie ou diagnostic.

32. Préparation pharmaceutique selon la revendication 30, destinée à une utilisation pour une administration intranasale.

33. Préparation pharmaceutique selon la revendication 30, destinée à une utilisation pour une administration orale.

34. Co-micelle selon l'une quelconque des revendications 9-29, **caractérisée en ce que** ladite co-micelle possède une taille en diamètre comprise entre 25 et 42 nm.
